# EUROPEAN PATENT APPLICATION

(11) **EP 4 599 679 A1**
(43) Date of publication of application: **13.08.2025**
(21) Application number: 25155915.9
(22) Date of filing: 05.02.2025
(51) Int. Cl.: A01N 63/40, A01P 1/00

(54) **METHOD OF PRODUCTION OF A COMBINATION OF PHAGES FOR BACTERIAL PHYTOPATHOGEN CONTROL**

(30) Priority: 09.02.2024 US 202463551857 P; 29.11.2024 US 202418963880
(71) Applicant: Exacta Bioscience, Inc., Lewes, Delaware 19958 (US)
(72) Inventor: Retamales Lara, Julio, Huechuraba (CL); Bustos Aguirre, José Manuel, Ciudad de Santiago (CL)
(74) Representative: Capitán García, Maria Nuria

(57) **Abstract**

A method is described for selecting bacteriophages for the biological control of infections in plants, trees, or plant pathogens, which includes several stages. First, a geographical area is identified with nearby water sources close to crops of interest. Then, soil or water samples are collected, and bacteriophages are amplified in two rounds using an appropriate culture medium. Using the double-layer agar technique, bacteriophages with lytic activity against plant pathogens are selected, ensuring no harm to beneficial bacteria. The synergy or cross-resistance between the selected strains is evaluated, and at least 2 or 3 strains showing lytic and/or enzymatic activity are chosen. Additionally, a preventive and curative treatment is outlined, applying the bacteriophages at key phenological stages of the crop. A consortium of bacteriophages with specific identifications obtained through this method is also mentioned.

## Description

### TECHNICAL FIELD

The invention is framed in the agricultural technical field because the control of a plague is sought and in the field of biotechnology by the proposed method.

### BACKGROUND AND PRIOR ART

There are multiple phytopathogenic pests of different origins that cause diseases to plants, trees, vegetables, whether of bacterial, fungal, or other origin. The invention addresses the problem of control and / or treatment of diseases generated by infections avoiding the generation of resistance of phytopathogens to treatment.

Throughout this document will be considered phytopathogens to exemplify the invention, however, this does not represent a limitation of the scope of protection.

One of the phytopathogens that serve to exemplify the invention is *Pseudomonas syringae* that affects different cultures causing, for example, bacterial cancer in cherry trees, blossom blast in pears, bacterial speck in tomatoes and bacterial leaf blight in blueberry, as well as affecting other cultures such as peaches and apricots. Other important bacteria that have a negative effect are *Xanthomonas (X. arboricola pv. Juglandis, X*. *erboricola pv. Corylina, X. arboricola pv. Pruni, X*. *campestris pv. Campestris, X*. *vesicatoria pv. Vesicatoria, X. avellanae), Pseudomonas (P. syringae pv. tomato, P. viridiflava) Clavibacter michiganensis, Ralstonia solanacearum, Pectobacterium carotovorum, Erwinia amylovora, Agrobacterium tumefaciens.*

*P. syringae* resists winters in infected plant tissues, which then in the spring or summer seasons will remain on the leaves, where the infection will continue without generating the disease. The bacteria then penetrate the leaves to eventually cause the disease. Evidence of the disease is reflected in necrotic spots on leaves or woody tissues, where the production of polysaccharides allows the bacteria to adhere and form biofilms that make it difficult to control. Finally, the phytopathogen in its next cycle explodes and produces the damage generating abnormal growths.

Some of the common problems associated with controlling *Pseudomonas syringae* infections include:
1. Antibiotic resistance: The bacteria can develop antibiotic resistance, limiting the effectiveness of traditional treatment methods.
2. Environmental persistence: *Pseudomonas syringae* can survive in various environmental conditions, making it difficult to eradicate infected areas.
3. Host Rank: The pathogen has a wide range of hosts, infecting numerous plant species, which complicates control efforts.
4. Spread of diseases: The bacteria can spread through irrigation water, rain and wind, leading to widespread infections in agricultural environments.

Regarding the above, the solutions currently available to control infections by *Pseudomonas syringae* include cultural practices, where the implementation of proper sanitation, crop rotation and pruning practices can help reduce the incidence of disease; biological control, where some beneficial microorganisms can suppress the growth of *Pseudomonas syringae,* and use them as biopesticides can be an environmentally friendly approach; chemical control, in which certain biological and copper-based pesticides are used to control *Pseudomonas syringae* infections. However, this approach must be carefully regulated due to environmental concerns; genetic resistance, in which the development and planting of genetically resistant plant varieties can be an effective long-term strategy to combat the disease; integrated Pest Management (IPM) with the combination of multiple control methods, including cultural practices, biological control, and selective use of pesticides, you can improve overall disease management.

It is essential to keep in mind that the availability and effectiveness of these solutions may vary depending on the specific strains of *Pseudomonas syringae* and regional factors. Therefore, research continues, and adaptation strategies are crucial to stay at the forefront of controlling this plant pathogen effectively.

Below is a summary of the previous art relating to the present invention:
WO2011014693A2 describes modified bacteriophages expressing specific peptides with antibacterial activity. Specifically, the document mentions *P. syringae* as one of the bacteria that can be combated with the bacteriophages described in the publication.

It should be noted that this document is very broad in its description and includes medical applications, such as for the treatment of Alzheimer's, and for the control of infections in plants.

WO02086072A2 describes specific polypeptides with antimicrobial activity, specifically against phytopathogens. More specifically, it is indicated that polypeptides are isolated from hemolymph from insect larvae that have been infected with pathogenic fungi for plants. The mention of bacteriophages is because it is listed as one of the vector alternatives to transform bacteria and produce the peptide with antimicrobial activity. In addition, there are published 7 related documents, which focus on different polypeptides each document.

US2020354689A1 describes modified bacteriophages that may be specific to certain bacteria, although there is no specific mention of the treatment of plant infections.

US2020254035A1 describes phages modified to fight bacterial infections. Among the pathogenic bacteria mentioned, *P. syringae* is indicated. The document is also very general since it mentions that as subjects of treatment can be chosen humans, animals, or plants.

US2015050717A1 describes bacteriophages for the control of bacterial infections in plants. It is specified that bacteriophages are selected depending on the expressed antibacterial peptides (Indolicidin, CecropinP1, Dermaseptin, Ponericin W1, Ponericin W3, Ponericin W4, Ponericin W5, Ponericin W6).

US2010322903A1 describes bacteriophages that express antimicrobial agents. The document is written in a general way, so they appear as subjects of application humans, animals, and plants. Specifically, one section mentions *P. syringae* as one of the bacteria to be fought.

**Microorganisms 2019, 7(9), 286** describes in vitro evidence of the effects of phage phi6 against *P*. *syringae.*

Applied Microbiology and Biotechnology volume 104, pages1319-1330 (2020**)** describes *in vitro* tests using phage phi6 to inactivate *P. syringae.* Specifically, tests are performed on kiwi leaves.

**PHAGE. Dec 2020.245-250** describes 3 isolated strains of phages targeted against several strains of *P. syringae.* Specifically, the strains were isolated from organic waste obtained from a treatment plant.

Applied biotechnology international, Volume 13, Issue 5, Thematic Issue on Agricultural Biotechnology, September 2020, Pages 1428-1445 describes the isolation of more than 70 phages from natural environments for use in the control of *P. syringae* in cherry trees.

As can be seen, the use of bacteriophages for the control of phytopathogens has been explored, however, the problem of generating resistance to bacteriophages remains. This invention proposes as a solution a method for the selection of specific bacteriophages phytopathogens that do not affect other bacteria beneficial to crops, while ensuring that the long-term resistance is avoided.

### SUMMARY OF THE INVENTION

The invention includes the development of a bacteriophage technology for control of Bacterial diseases caused by phytopathogens. In the development to demonstrate the effectiveness of the method, three bacteriophages from natural sources were selected and their ability to destroy different strains of *Pseudomonas syringae* was demonstrated. *In vitro* efficacy tests showed that bacteriophages eliminate 99% of the bacterial population within an hour. In the tests of effectiveness *in plant,* the treatment with bacteriophages showed a preventive and curative effect against bacterial cancer in cherry trees.

### BRIEF DESCRIPTION OF FIGURES

Figure 1: The three selected bacteriophages have clear or transparent lysis halo, demonstrating lytic activity on *P. syringae* pv. *syringae* (Pss). Moreover, phages RQ1 (RQ1 (International Depositary Authority of Canada, IDAC, accession number 281124-03), and C1 (IDAC accession number 281124-01) have enzyme activity in addition to cell lytic activity. This image shows synergy between bacteriophages for the control of bacteria since some bacteriophages due to the presence of specific enzymes may facilitate the work of other bacteriophages that do not have them.
Figure 2: Multiple sequence alignment for lytic enzymes in selected phages.
Figure 3: Shelf-life of bacteriophages formulated and non-formulated product (squares formulated, circles non-formulated, x-axis days).
Figure 4: genome-wide phylogenetic tree was made. Phages were compared to the top 10 blast hits per phage (30 phages total).
Figure 5: Bacterial resistance to bacteriophages combinations.
Figure 6: When there is a variation in the multiplicity of infection (MOI), a bacteriophage significantly decrease its lytic activity against *Pss* in an *in vitro* culture. However, this bacteriophage being mixed with 1 or 2 different bacteriophages and with the presence of other enzymes/protein reverses its effect. This represents the synergy between the different phages, since the combination of the different enzymes or other metabolites produced by each of them allows a combination that is not possible to obtain with each of them separately.
Figure 7: Pss bacteriophage synergy in vivo. Time in hours.

### DETAILED DESCRIPTION OF THE INVENTION

1. Firstly, the invention corresponds to a method of selecting bacteriophages for biological control by means of preventive and curative treatment in infections in plants, trees, or vegetables phytopathogens where the selection of bacteriophages is made looking for the synergistic effect of their combination and consequently avoid the appearance of resistance to the proposed treatments.
2. In a second aspect, the strains of bacteriophages selected according to the bacteriophage selection method of the present invention are considered.
3. In a third aspect a preventive and curative treatment method against infections of phytopathogens is contemplated using the phages selected with the method of invention.

In a particular realization and as a first aspect, the method of selecting bacteriophages includes the following steps:
- Determine a geographical area with water sources close to crops of interest;
- Obtain samples from the soil, water sources and/or plant tissue identified in the previous step;
- Generate first round of amplification of bacteriophages using samples, previously treated with an organic solvent (e.g. chloroform, ethanol, isopropanol), using host bacteria (*virulent strain of Pss, Pseudomonas syringe pv. morsprunorum, Pseudomonas syringe pv. actinidae, Pseudomonas syringe pv. tomato, Pseudomonas gramini* or similar), *Xanthomonas (X. arboricola pv. Juglandis, X. erboricola pv. Corylina, X. arboricola pv. Pruni,* X. *campestris pv. Campestris, X*. *vesicatoria pv. Vesicatoria, X. avellanae), Pseudomonas, P. viridiflava), Clavibacter michiganensis, Ralstonia solanacearum, Pectobacterium carotovorum, Erwinia amylovora, Agrobacterium tumefaciens* and grown in a microbiological nutrient culture medium is required (based on tryptones, peptones and/or yeast extracts, for example, Luria Bertani or LB (2x) for a maximum of 24 hours at 25°C and 150 rpm of stirring.
- Generate a second round of amplification of bacteriophages using a filtration of the previous culture (0.22 µm) with a host strain known to allow the multiplication of bacteriophages, to be chosen and under the same operating conditions described above.
- Obtain individual lysis plates using the double-layer agar technique, discarding those that have turbid-lysis plates, since they will be considered preliminary as lysogenic phages.
- Select strains of bacteriophages that have lytic activity against phytopathogen, with or without enzymatic activity against this bacterium, and that do not have lytic activity against beneficial bacteria.
- Determine the resistance or cross-synergy of the mixture of bacteriophage strains.
- Select at least 2 or preferably 3 or more bacteriophages that have lytic and/or enzyme lytic activity against phytopathogens and that do not have lytic activity against beneficial bacteria and where at least 2 or preferably 3 selected bacteriophages present resistance or cross-synergy.

Regarding the second aspect of the invention, the strains selected with the previous method have been deposited in the International Depository Authority of Canada (IDAC).

Finally, in the third aspect of the invention, the protocol for preventive treatment or curative against phytopathogens includes the following steps:
1. Dilution of the product according to the crops to be treated with a concentration from 10⁵ to 10⁹ UFP/ml.
2. Foliar, root or substrate application, by spraying, irrigation and/or immersion, during flowering and/or other phenological stage of the plant that involves a high bacterial load.
3. Apply a minimum of 2 times, preferably 3 or more times with intervals of 7-15 days.

### EXAMPLES

### (A) Detection of differentiating parameters of bacteriophages:

### 1) Range of hosts:

Methodology: Through a spot test using the double-layer agar technique, previously isolated bacteriophages were evaluated in their ability to destroy different representatives of bacterial phytopathogens used as indicators. The bacteriophages selected according to the method of invention were called RQ1 (International Depositary Authority of Canada, IDAC, accession number 281124-03), C1 (IDAC accession number 281124-01) and D3 (IDAC accession number 281124-02). These bacteriophages have specific activity against the group *Pseudomonas syringae,* without causing the lysis death of other bacteria such as *Xanthomonas, Curtobacterium, Erwinia* and *Pectobacterium,* included in the test. In addition, using the same methodology, the mixture of the three bacteriophages indicated has the ability to produce the bacterial lysis of different pathogenic representatives of the Ps group, both in *Pseudomonas syringae pv. syringae (Pss)* (accession number 281124-04 at the International Depository Authority of Canada (IDAC)), *Pseudomonas syringae pv. morsprunorum (Psm) and Pseudomonas syringae pv. actinidiae (Psa).*

### 2) Presence of lytic activity and action of other lysis enzyme/protein.

Methodology: To determine the lytic activity of bacteriophages selected from the previous test, the double-layer agar technique was developed using a Pss isolate as a strain indicator. In this case, isolated lysis plates were photographed to determine the clarity of the halo, demonstrating lytic activity and ruling out unwanted lysogenic activity (preliminary analysis). From this photographic analysis, the presence of other enzymes/protein activity was determined by forming a second halo around the central lysis halo. Result: The three selected bacteriophages have clear or transparent lysis halo, demonstrating lytic activity on Pss. Moreover, phages RQ1 and C1 have activity of second enzymes activity in addition to lytic activity.

Result: the three selected bacteriophages presented clear or transparent lysis halo, which suggests the presence of lytic activity on Pss. Subsequent genomic analyses generated with the PhageAI software confirm the absence of related genes related to lysogeny, antibiotic resistance and virulence, so it is possible to confirm the lytic nature of the phages. Moreover, the RQ1 and C1 phages show hydrolytic activity on the Pss culture (indicated by a red arrow), which is imperceptible with the bacteriophage D3.

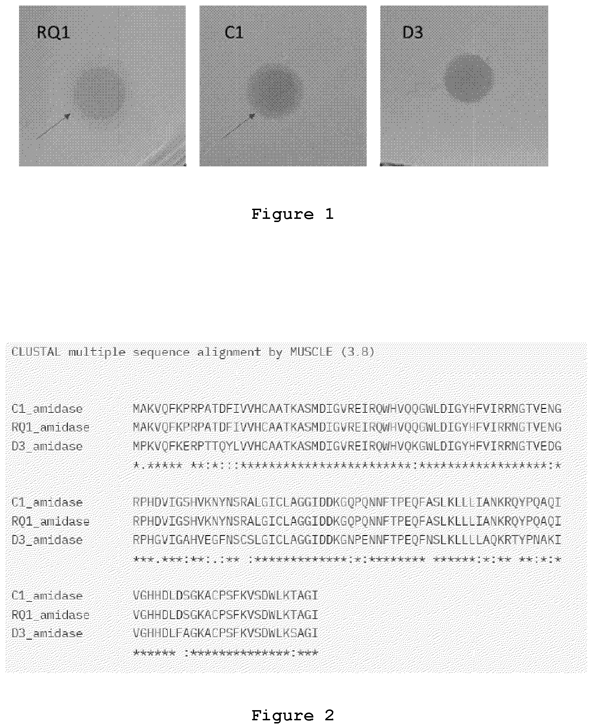

The selected bacteriophages do not have genes that encode depolymerases, however, all three phages have genes that encode endolysins (N-acetylmuramoyl-L-alanine amidase; Pfam PF01510.25). The amino acid sequences of these endolysins were aligned with MUSCLE 3.8 (https://www.ebi.ac.uk/jdispatcher/msa/muscle). As a result, the amino acid sequences of C1 and RQ1 were shown to be identical, while the D3 sequence differs from these with a low identity (82.19) (Figure 2).

### 3) Shelf-life stability:

Methodology: To determine the shelf life of bacteriophages, tests were carried out using a stock of 1X10⁵ UFP/mL of a phage cocktail between RQ1, C1 and D3 and the addition of co-formulants based on i) 1-3% thickening agents (celluloses, xanthan gum, alginate or other polysaccharides; ii) 1-5% dispersants (celluloses and polyvinylpyrrolidone), iii) 3-8% humectants (glycerol, polyethylene glycol, ethylene glycol and iv) UV protectants (zinc oxide, titanium oxide). These phages, in triplicate, were arranged in test tubes at 25°C for 5 months. The variation of UFP/mL was evaluated from time to time compared to a bacteriophage stock in the absence of co-formulants.

The addition of co-formulant to a bacteriophage stock allows to maintain for at least 5 months the shelf life of a product based on these bacteriophages, which projects a minimum period of 12 months of final shelf life.

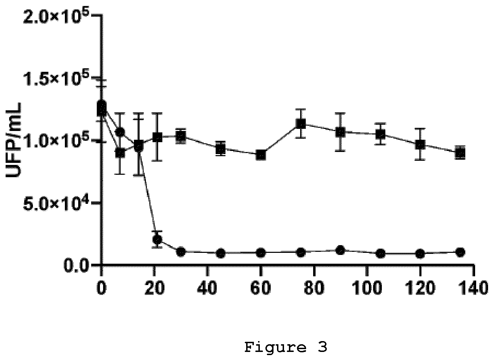

Results: As shown in the attached graph (Figure 3), the addition of co-formulant to a bacteriophage stock allows the shelf life of a product based on these bacteriophages to be maintained for at least 5 months, which projects a minimum period of 12 months of final shelf life.

### 4) Genomic analysis of bacteriophages:

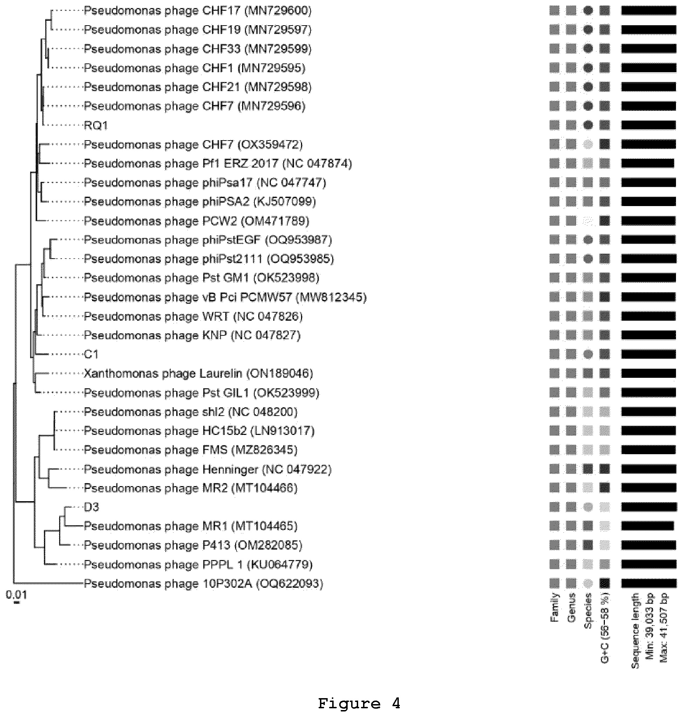

Methodology: To show that the selected bacteriophages are grouped into different phylogenetic groups, comparative genomics studies were carried out. To do this, the genetic material of each selected bacteriophage was initially obtained, being sequenced using the Ilumina platform by the company MicrobesNG (United Kingdom). The information obtained was processed with the Geneious Prime bioinformatics software to annotate structural genes and enzymes that are associated with the lytic processes of each virus. To study the intergenomic similarity of bacteriophages, the VIRIDIC platform (Virus Intergenomic Distance Calculator; https: Taxonomic prediction was performed with PhageAI. Using the VICTOR (Virus Classification and Tree Building Online Resource; https://ggdc.dsmz.de/victor.php) tool, a genome-wide phylogenetic tree was made. Phages were compared to the top 10 blast hits per phage (30 phages total) (Figure 4). These tools were used because they are developed for the analysis of viral genomes.

Results: The results of the PhageAI tool suggest that phages belong to the order Caudoviricetes, family Autographiviridae and genus Ghunavirus. On the other hand, the bacteriophages analyzed showed intergenomic similarities that can be seen in Table 2. According to the standards of the International Committee on Taxonomy of Viruses (ICTV), phages belong to three different species.

| **Fago** | **C1** | **RQ1** | **D3** |
|---|---|---|---|
| **C1** | 100 | 94.649 | 63.728 |
| **RQ1** | 94.649 | 100 | 63.548 |
| **D3** | 63.728 | 63.548 | 100 |

The RQ1 phage was found close to CHF phages, which were previously isolated in Chile as a potential biocontrol against Pseudomonas syringae pv. actinidiae (Flores et al. 2020), with similarities ranging from 96,553 to 99,112. According to the ICTV recommendations, RQ1 would belong to a variant of this same species. On the other hand, bacteriophage C1 is close to phages KNP (NC_047827.1; similarity 95.006) and WRT (NC_047826.1; similarity 93.944), phages isolated in Poland that infect Pseudomonas fluorescens (Nowicki et al. 2017). Bacteriophage D3 has a similarity of 92,593 with phage P413 (OM282085.1), which infects Pseudomonas savastanoi pv. glycinea (Psg), the causative agent of bacterial spot in soybeans (Tarakanov et al. 2022). Phage D3 also possesses a similarity of 87.617 to phage MR1 (MT104465.1), which was previously used in phage biocontrol to combat disease-causing Pseudomonas syringae pathogens in cherry plants (Rabiey et al. 2020). Therefore, the phylogenetic tree generated by VICTOR allows us to conclude that the three bacteriophages selected are from different species, which justifies their location in different clusters.

### B) Selection of bacteriophage cocktail:

### 1) Cross resistance (cross synergy)

Methodology: With the idea of knowing the emergence of bacteriophage-resistant Pss variants, two assays were carried out to determine the synergistic effect of bacteriophages in reducing the in vitro appearance of bacteriophage-insensitive mutants (BIMs). To do this, initially the bacteriophages selected individually as a mixture were added in flasks to a MOI of 100 and left in co-culture for 20 minutes. After this time, serial dilutions were carried out and the colonies resistant to Pss bacteriophages (CFU/mL) were quantified in microbiological agar plates, which contained individual bacteriophages or in mixture, as appropriate to the treatment, to ensure resistance. All assays were performed in triplicate and purchased with bacterial cultures of Pss without the addition of bacteriophages. Subsequently, and to confirm the synergy results, colonies were randomly isolated from the BIM assays and plate lysis assays were performed with these bacteria, evaluating the ability of other bacteriophages to be able to lyse it in vitro (double agar layer), thus determining a range of lytic action against Pss populations (intraspecific host range).

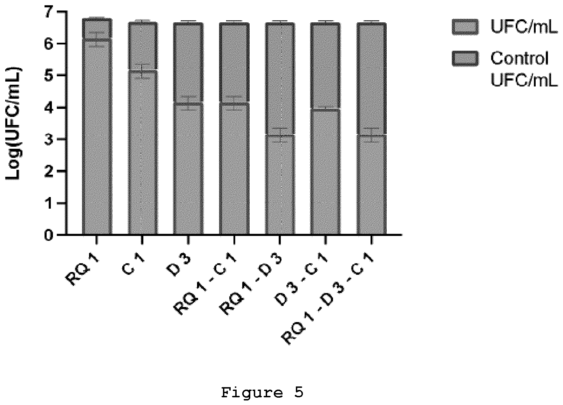

On the other hand, to confirm the synergistic effect of these bacteriophages, cross-lysis assays were performed. As shown in the attached table, it is possible to determine which bacteriophage-resistant bacteria can be lysed by other bacteriophages included in the study or cocktail. For example, RQ1. R1 isolated from Rq1 phage resistant Pss can be lysed by C1 and/or D3 phages (Figure 5).

| Pss resistant to a specific bacteriophage | Phage RQ1 | Phage C1 | Phage D3 |
|---|---|---|---|
| RQ1.R1 | X | ✔ | ✔ |
| RQ1. R3 | X | ✔ | X |
| RQ1.R4 | X | ✔ | X |
| RQ1.R8 | X | X | ✔ |
| RQ1.R10.1 | X | ✔ | X |
| C1.R2 | ✔ | X | ✔ |
| C1.R3 | X | X | ✔ |
| C1.R5 | ✔ | X | X |
| D3.R1 | ✔ | ✔ | X |
| D3. R5 | ✔ | ✔ | X |
| D3.R6 | ✔ | X | X |
| D3.R7 | X | | X |
| D3. R8 | X | ✔ | X |
| D3.R9 | ✔ | ✔ | X |

| | | | |
|---|---|---|---|
| **✔: with lytic effect** **X: without lytic effect** | | | |

### C) Effectiveness in vitro model (synergy)

Methodology: The objective of this methodology is to evidence a control of Pss in in vitro cultures in a synergistic way among the selected bacteriophages by observing the decrease in the optical density of the culture. In this sense, the time taken by the total lysis of Pss in the presence of individual phages or in mixture will be determined. To do this, three values of Phage Infection Multiplicity (MOI: 10, 1 and 0.1) will be applied in liquid cultures of Pss at flask scale. The kinetics of bacterial lysis with the treatments of individual or mixed phages (RQ1, RQ1+C1, RQ1+D3, C1+D3, RQ1+C1+D3) will be compared with Pss cultures in the absence of virus. Results: The graphs obtained show that the RQ1 and C1 bacteriophages individually or in mixture present a similar pattern in the kinetics of bacterial decay of Pss regardless of the MOI applied. On the contrary, as the MOI of application specifically decreases to the lytic bacteriophage D3, its ability to eliminate the bacterial population of Pss during the period evaluated decreases proportionally. However, when this bacteriophage is arranged in mixture with RQ1 and C1, the behavior is reversed, drastically reducing the growth of Pss in vitro.

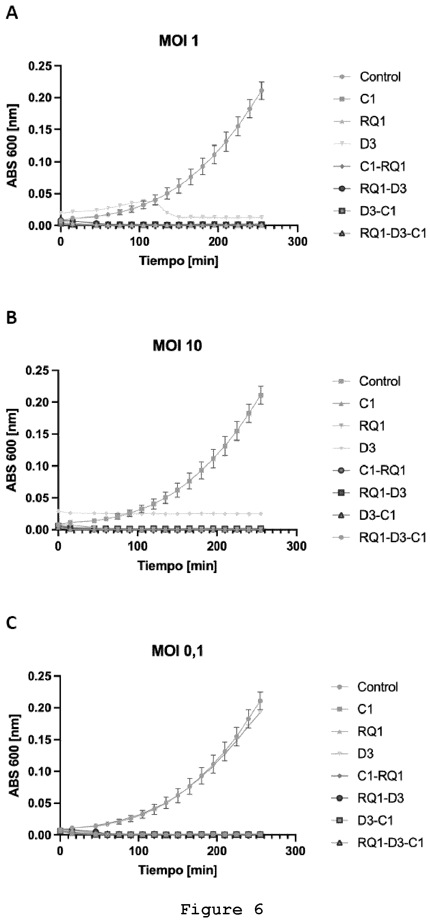

Methodology: The objective of this methodology is to evidence a control of Pss in in vitro cultures in a synergistic way among the selected bacteriophages by observing the decrease in the optical density of the culture. In this sense, the time taken by the total lysis of Pss in the presence of individual phages or in mixture will be determined. To do this, three values of Phage Infection Multiplicity (MOI: 10, 1 and 0.1) will be applied in liquid cultures of Pss at flask scale. The kinetics of bacterial lysis with the treatments of individual or mixed phages (RQ1, RQ1+C1, RQ1+D3, C1+D3, RQ1+C1+D3) will be compared with Pss cultures in the absence of virus. Results: The graphs obtained show that the RQ1 and C1 bacteriophages individually or in mixture present a similar pattern in the kinetics of bacterial decay of Pss regardless of the MOI applied. On the contrary, as the MOI of application specifically decreases to the lytic bacteriophage D3, its ability to eliminate the bacterial population of Pss during the period evaluated decreases proportionally. However, when this bacteriophage is arranged in mixture with RQ1 and C1, the behavior is reversed, drastically reducing the growth of Pss in vitro. (Figure 6)

### E) Effectiveness in plant model (synergy)

Methodology: The objective of this methodology is to evidence a control of Pss in in vivo models on tomato plants, to evidence the synergistic effect of the selected bacteriophages. In this sense, the effectiveness of bacteriophages in reducing the Pss load (CFU/mL), applied individually and in mixture, was determined. To this end, bacteriophages will be applied at a final MOI of 0.1, on plants previously inoculated by spraying with Pss (1×10⁶ CFU/mL). For the purposes of this study, tomato control plants inoculated with Pss and absolute wet controls will be included. All results were analyzed in triplicate, in a climatic chamber under constant control of temperature (20°C), Relative Humidity (80%) and photoperiod (16 hours light/8 hours darkness). The results were analyzed using the One-Way ANOVA test.

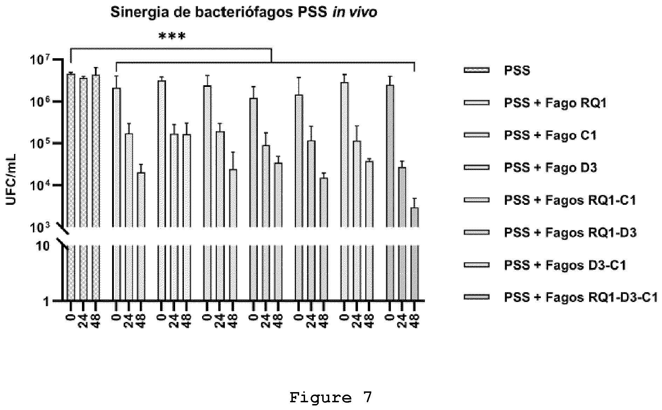

Result: As expected, the groups inoculated with Pss (control) have counts that exceed 10⁶ CFU/mL of Pss up to 48 hours of exposure. Individual bacteriophages can reduce the bacterial load by almost 2 orders of magnitude at the end of the 48 hours of analysis. Similar results were obtained with phages mixed in pairs. However, the addition of the three bacteriophages together achieves a reduction in Pss of almost three orders of magnitude, which confirms the synergy of the bacteriophage mixture in the reduction of the bacterial load of Pss in plant models (Figure 7).

### Identification of deposited bacteriophages of the invention

The microorganisms part of the invention were deposited before the International Depositary Authority of Canada with the following accession numbers:
RQ1 IDAC accession number 281124-03.
C1 IDAC accession number 281124-01.
D3 IDAC accession number 281124-02.
*Pseudomonas syringae pathovar syringae,* also mentioned in this document as *Pseudomonas syringae Pss* accession number 281124-04.

### INDUSTRIAL APPLICATION

The present invention has industrial agricultural and biotechnological applications.

## Claims

1. A method of selecting bacteriophages for biological control by means of preventive and curative treatment in infections in plants, trees, or vegetable phytopathogens where the selected bacteriophages have a synergistic effect thus avoiding the appearance of resistance to proposed treatments, wherein the method comprises the following steps:
a) Determine a geographical area with water sources close to crops of interest;
b) Obtain samples from the soil or water sources identified in the previous step;
c) Generate first round of amplification of bacteriophages using samples, previously treated with an organic solvent (chloroform, ethanol, isopropanol), using host bacteria and grown in a proper microbiological nutrient culture medium is required (based on tryptones, peptones and/or yeast extracts) ;
d) Generate a second round of amplification of bacteriophages filtering the previous culture;
e) Obtain individual lysis plates using the double-layer agar technique, discarding those that have turbid-lysis plates, since they will be considered preliminary as lysogenic phages;
f) Select strains of bacteriophages that have lytic activity against phytopathogen, with or without enzymatic activity against this bacterium, and that do not have lytic activity against beneficial bacteria;
g) Determine the resistance or cross-synergy of the mixture of bacteriophage strains;
h) Select at least 2 or preferably 3 or more bacteriophages that have lytic and/or enzyme lytic activity against phytopathogens and that do not have lytic activity against beneficial bacteria and where at least 2 or preferably 3 selected bacteriophages present resistance or cross-synergy.

2. The method of selecting bacteriophages according to claim 1, wherein the host bacteria are *virulent strain of Pseudomonas syringae pv. syringae, Pseudomonas syringe pv. morsprunorum, Pseudomonas syringe pv. actinidiae, Pseudomonas syringe pv. tomato, Pseudomonas gramini, Xanthomonas (X. arboricola pv. Juglandis,* X. *erboricola pv. Corylina, X. arboricola pv. Pruni,* X. *campestris pv. Campestris,* X. *vesicatoria pv. Vesicatoria, X. avellanae), Pseudomonas (P. viridiflava), Clavibacter michiganensis, Ralstonia solanacearum, Pectobacterium carotovorum, Erwinia amylovora, Agrobacterium tumefaciens* or other bacterial phytopathogen.

3. The method of selecting bacteriophages according to claim 1, wherein the organic solvent is chloroform.

4. The method of selecting bacteriophages according to claim 1, where a microbiological nutrient culture medium is required (based on tryptones, peptones and/or yeast extracts) and maintained for a maximum of 24 hours at 25°C and 150 rpm of stirring.

5. The method of selecting bacteriophages according to claim 1, where in the filtering is made with a 0.22 µm in half LB with a host strain known to allow the multiplication of bacteriophages, to be chosen and under the same operating conditions described above.

6. A preventive and curative treatment method against infections of phytopathogens is contemplated using the phages selected with the method of claims 1 to 5, wherein the preventive and curative method comprises the following steps:
a) diluting the product according to the crops to be treated with a concentration from 10⁵ to 10⁹ UFP/ml;
b) providing foliar, roots or substrate application, by spraying or immersion, during flowering and/or or other phenological stage of the plant that involves a high bacterial load;
c) Apply the foliar application for a minimum of 2 times, preferably 3 or more times with intervals of 7-15 days.

7. Consortium of bacteriophages obtained using the method of claims 1 to 5, wherein the bacteriophages are:
RQ1 IDAC accession number 281124-03;
C1 IDAC accession number 281124-01; and
D3 IDAC accession number 281124-02.

8. Consortium of bacteriophages obtained using the method of claims 1 to 5, wherein the host bacteria for the selection of bacteriophages according to claims 1 to 5 is *Pseudomonas syringae pathovar syringae,* also mentioned in this document as *Pseudomonas syringae Pss* IDAC accession number 281124-04.
